# EUROPEAN PATENT SPECIFICATION

(11) **EP 1 388 344 B1**
(45) Date of publication and mention of the grant of the patent: **12.03.2014**
(21) Application number: 03017260.5
(22) Date of filing: 30.07.2003
(51) Int. Cl.: A61K 31/045, A61K 36/185, A61K 36/258, A61K 36/537, A61K 36/54

(54) **Composition comprising Salvia Miltiorrhiza, Ginseng, Borneol for treating heart disease, and cerebrovascular diseases**
Zusammensetsung enthaltend Salvia Miltiorrhiza, Ginseng, Borneol zur Behandlung von zerebrovaskulären und Herzkrankheiten
Composition comprenant Salvia Miltiorrhiza, Ginseng, Borneol pour le traitement des maladies cardiaques et cerebrovasculaires

(30) Priority: 31.07.2002 US 210548
(43) Date of publication of application: 11.02.2004
(73) Proprietor: Tasly Pharmaceutical Group Co., Ltd., Tianjin 300410 (CN)
(72) Inventor: Yan, Xijun, Tianjin (CN); Wu, Naifeng, Tianjin (CN); Guo, Zhixin, Tianjin, P.R.China (CN); Ye, Zhengliang, Tianjin,P.R.China (CN); Liu, Yan, Tianjin (CN)
(74) Representative: Lecca, Patricia S.

(56) References cited:
- WO-A-02/058625
- CN-A- 1 337 257
- US-A- 5 589 182
- LI R ET AL: "Quantitative determination of total tanshinone in salvia miltiorrhiza tablet compound" YIYAO GONGYE - PHARMACEUTICAL INDUSTRY, SHANGHAI, CN, vol. 17, no. 11, 1986, pages 513-514, XP002954785 ISSN: 0255-7223
- NI K ET AL: "Determination of active components of salvia miltiorrhiza and notoginseng in compound salvia miltiorrhiza tablets by TLC-densitometry" YAOWU FENXI ZAZHI - CHINESE JOURNAL OF PHARMACEUTICAL ANALYSIS, ZHONGGUO YAOXUEHUI, BEIJING, CN, vol. 9, no. 2, 1989, pages 74-77, XP002954784 ISSN: 0254-1793
- DATABASE MEDLINE [Online] US NATIONAL LIBRARY OF MEDICINE (NLM), BETHESDA, MD, US; March 1990 (1990-03) LIU Y ET AL: "[Evaluation of radix Salviae Miltiorrhizae and its preparation]" Database accession no. NLM2085402 XP002262545 & ZHONGGUO ZHONG YAO ZA ZHI = ZHONGGUO ZHONGYAO ZAZHI = CHINA JOURNAL OF CHINESE MATERIA MEDICA. CHINA MAR 1990, vol. 15, no. 3, March 1990 (1990-03), pages 159-162, 190, ISSN: 1001-5302
- ANONYMOUS: INTERNET ARTICLE, [Online] XP002262543 Retrieved from the Internet: <URL:http://www.carbotrading.com/Products/ Medicine/JZ016.htm> [retrieved on 2003-11-18]
- ANONYMOUS: INTERNET ARTICLE, [Online] XP002262544 Retrieved from the Internet: <URL:http://www.craneherb.com/products/pro duct001513> [retrieved on 2003-11-18]

## Description

Heart disease includes a number of conditions affecting the structures or function of the heart. They can include: coronary artery disease, including heart attack, abnormal heart rhythms, heart failure, heart valve disease, congenital heart disease, heart muscle disease or cardiomyopathy, and pericardial disease.

Heart disease is the leading cause of death for both men and women in the United States. Treatment of heart disease depends on the type of disease as well as many additional factors. Coronary artery disease is treated with: medications such as aspirin; beta-blockers; nitroglycerin tablets, spray, and patches; calcium channel blockers; thrombolytic therapy; and surgeries such as coronary angioplasty and coronary bypass operations.

The number of patients with cardiovascular or cerebrovascular disease increases along with higher living standards (better food supply), the worldwide aging problem, and young adult's involvement. It has become the second most-common disease worldwide threatening the health of human beings.

Angina pectoris is caused by insufficient blood and oxygen supply to the heart. The main clinical symptom is chest pain. It is caused by atherosclerosis or spasms of the coronary artery in about 90% of the angina pectoris patients.

The major treatments for angina pectoris are vessel dilation, lowering of blood viscosity, anti-aggregation of platelets and anti-coagulation. The traditional medicines used are nitrates, beta-adrenoceptor blocking drugs and calcium-channel blocking drugs. However, all these drugs have many side effects which make them unsuitable for long-term use. For example, patients experience a swelling sensation in their heads, rapid heartbeat and even coma after taking glyceryl trinitrate.

This invention involves a medication which can prevent and cure coronary heart disease with angina pectoris, the methods of manufacture and other usages of the medication. The medication, Dan Shen Pill (DSP), is made from a variety of Chinese herbs using a series of standard procedures. To this regard we may cite the Chinese application CN1337257 which describes the use of DSP.

DSP is an improvement on Dan Shen Tablet (DST) (recorded in Pharmacopoeia of the People's Republic of China in Edition, 1977, 1985, 1995, and 2000), but there are significant differences between DSP and DST: proportions in their formulas, manufacturing techniques and their clinical results.

Although there are many Chinese herbal medicines used for treating angina pectoris, fewer people use them nowadays. DST or capsules are being sold in the market, but their manufacturing techniques are old, their efficacy is low and there are no quality standards. For example, a process for preparing DSP comprising extracts of Panax Notoginseng, Radix Salviae Miltorrhizae, and borneol, wherein each of the water soluble components are extracted separately is described in the international patent publication WO02/058625.

DST is taken orally and absorbed in the gastrointestinal tract, where it is absorbed into blood vessels after processing in the liver. The bioavailability is low, and the absorption speed is low, which is not suitable for the emergency treatment of patients with angina pectoris. Accordingly, DSP is superior to DST.

### SUMMARY OF INVENTION

This invention provides a composition for treating heart disease. The heart disease includes chronic stable angina pectoris, coronary artery disease (including heart attack), abnormal heart rhythm, heart failure, heart valve disease, congenital heart disease, heart muscle disease (cardiomyopathy), and pericardial disease. This invention also provides a composition comprising herb extracts from raw herbs of 80.0-97.0% Radix Salviae Miltorrhizae, 1.0-19.0% Panax Notoginseng and 0.1-1.0 Borneol. This invention provides the above composition wherein the extracts are made from raw herbs of 90.0-97.0% Radix Salviae Miltorrhizae, 2.5-9.6% Panax Notoginseng and 0.2-0.5% Borneol. This invention provides the above composition wherein the extracts are made from raw herbs of 89.8% Radix Salviae Miltorrhizae, 9.6% Panax Notoginseng and 0.5% Borneol.

This invention provides a preparation method comprising steps of: (1) obtaining an appropriate amount of smashed Radix Salviae Miltorrhizae and Panax Notoginseng; (2) extracting the obtained Radix Salviae Miltorrhizae, and Panax Notoginseng in hot aqueous reflux; (3) filtering and combining the extracts to form a combined extract; (4) concentrating the combined extract from step (3) to an appropriate ratio of the volume of the concentrated extract to the weight of the inputted herbal materials to form a concentrated extract; (5) precipitation by organic solvent to form a precipitate; (6) concentrating the supernatant liquid of precipitate resulting from step (5) to form a plaster; (7) mixing the plaster from step (6) with an appropriate amount of Borneol, thereby producing the composition comprising extracts of Radix Salviae Miltorrhizae, Panax Notoginseng and Borneol for heart disease.

The above composition contains Sodium 3' 4-dihydroxyphenyllactate, 3' 4-dihydroxyphenyllactate, Protocatechuic Aldehyde, Salvianolic acid A, B, C, D, E, F, Rosemarinic acid, Ginsenoside Rg1, Ginsenoside Rb1, Ginsenoside Re, Notoginsenoside R1, Borneol, etc.

In another embodiment, the above composition further contains methyl tanshinonate, methyl rosmarinate, danshexinkum, lithospermic acid, d-borneol, 1- borneol, tanshinone I, tanshinone IIA, tanshinone IIB, tanshinone V, tanshinone VI, isotanshinone, miltirone, dihydrotanshinone, 1-dehydrotanshinone and neocryptotanshinone, Salvianolic acid G and Salvianolic acid I, lithospermic acid B, ethyl lithospermate, dimethyl lithospermate, monomethyl lithospermate, ginsenoside Rd, ginsenoside Rg2, ginsenoside Rg3, ginsenoside Rh1 and ginsenoside Rh2, notoginsenoside R2, notoginsenoside R3, notoginsenoside R4, notoginsenoside R6, and notoginsenoside R7.

This invention also provides a pill composition capable of treating chronic stable angina pectoris comprising about 0.14 to about 0.18 mg 3' 4-dihydroxyphenyllactate per pill, about 6.50 to about 40.50 mg Sanchinoside R1 per pill and about 25.60 to about 86.20 mg Ginsenoside Rg1 per pill.

This invention provides a composition for the treatment of cardiovascular and cerebrovascular disease comprising pure active ingredients extracted from *Salvia miltiorrhiza* Beg., *Panax notoginseng* or Ginseng, and *Dryobalanops aromatica Geartu.F.* or *Cinnammon camphor* or synthetic borneol. The active ingredient extracted from *Salvia miltiorrhiza* Beg. contains one or more ingredients selected from tanshinone, salvianolic acid, methyl tanshinonate, rosmarinic acid, methyl rosmarinate, danshexinkum, protocatechualdehyde, Sodium 3' 4-dihydroxyphenyllactate, lithospermic acid. The ingredient extracted from *Panax notoginseng* or Ginseng contains one or more ingredients selected from notoginsenoside and ginsenoside. The ingredient extracted from *Dryobalanops aromatica Geartu.F.* or *Cinnammon camphor.* contains d-borneol or 1- borneol or both of them.

The said tanshinone includes tanshinone I, tanshinone IIA, tanshinone IIB, tanshinone V, tanshinone VI, isotanshinone, miltirone, dihydrotanshinone, 1-dehydrotanshinone, and neocryptotanshinone. The said salvianolic acid includes Salvianolic acid A, Salvianolic acid B, Salvianolic acid C, Salvianolic acid D, Salvianolic acid E, Salvianolic acid G, and Salvianolic acid I. The said lithospermic acid includes lithospermic acid B, ethyl lithospermate, dimethyl lithospermate, and monomethyl lithospermate. The said ginsenoside includes ginsenoside Rb1, ginsenoside Rd, ginsenoside Re, ginsenoside Rg1, ginsenoside Rg2, ginsenoside Rg3, ginsenoside Rh1, and ginsenoside Rh2. The said notoginsenoside includes notoginsenoside R1, notoginsenoside R2, notoginsenoside- R3, notoginsenoside R4, notoginsenoside R6, notoginsenoside R7.

This invention provides the above composition comprising Magnesium Salvianolate B or 3' 4-dihydroxyphenyllactate extracted from the *Salvia miltiorrhiza* Beg., Ginsenoside Rb₁ extracted from the *Panax notoginseng* or Ginseng, and d-Borneol extracted from the *Dryobalanops aromatica Geartu.F.* or *Cinnammon camphor.* In an embodiment, the composition comprises 10-80mg of Magnesium Salvianolate B, 10-50mg of Ginsenoside Rb₁ and 5-30mg of Borneol. In a further embodiment, the composition is characterized by comprising 50mg of Magnesium Salvianolate B, 20mg of Ginsenoside Rb₁ and 15mg of d-Borneol.

This invention provides the above composition, comprising 5-80mg of sodium 3' 4-dihydroxyphenyllactate, 10-50mg of Ginsenoside Rb₁ and 5-30mg of Borneol. In a further embodiment, this invention provides the above composition comprising 20mg of sodium 3' 4-dihydroxyphenyllactate, 20mg of Ginsenoside Rb₁ and 15mg of d-Borneol.

This invention provides the above composition further comprising one or more kinds of excipients such as polyethylene glycol, xylitol, lactobacillus, starch for preparation of dripping pills, wherein the ratio of weight the of above total extracts or above total pure ingredients to the weight of excipient is 1:1-1:4. For example, 50mg of Magnesium Salvianolate B, 20mg of Ginsenoside Rb₁, 15mg of d-Borneol and 265mg of excipient are contained per ten dripping pills; 20mg of sodium 3' 4-dihydroxyphenyllactate, 20mg of Ginsenoside Rb₁ and 15mg of d-Borneol and 265mg of excipient are contained per ten dripping pills.

The Salvianolate B was extracted by steps of: Pulverizing *Salvia miltiorrhiza* Beg. into a fine powder, and decocting twice with hot water; Combining the decoctions, and concentrating under vacuum at about 50°C; Loading the solution from the step (b) into macroporous adsorption resin and, after washing with water, eluting column with 40% ethanol; After recovering the ethanol from solution obtained in the step (c), refining it by Sephadex LH-20 or other gel columns with the similar characteristics, eluting with ethanol and collecting eluant containing Magnesium Salvianolate B; Repeating the process of step (d) until the concentration of Magnesium Salvianolate B reaches more than 90%. The purity of concentration of Magnesium Salvianolate B was assayed by HPLC at a detective wavelength of 281 nm.

The Ginsenoside Rb₁ was extracted by steps of: Pulverizing *Panax notoginseng* or *Ginseng* into fine powder, and decocting with water; or refluxing with 70% ethanol; or percolating with 70% ethanol; Recovering solvent from the solution at reduced pressure; Loading the solution from the step (b) into macroporous adsorption resin, and after washing with water, eluting column with 40% ethanol; After recovering the ethanol from solution obtained in the step (c), refining it by silica gel column; Eluting the column with chloroform, methanol and water in the ratio of 6:3:1, respectively, and collecting eluant; Using TLC for examination of Ginsenoside Rb₁ and recovering the solvent, thereby obtaining Ginsenoside Rb₁.

### DETAILED DESCRIPTION OF THE INVENTION

This invention provides a process for preparing a composition for treating heart disease. The heart disease includes chronic stable angina pectoris, coronary artery disease (including heart attack), abnormal heart rhythm, heart failure, heart valve disease, congenital heart disease, heart muscle disease (cardiomyopathy), and pericardial disease.

More particularly, the present invention relates to a process for preparing a composition for treatment of chronic stable angina pectoris, characterized in comprising the steps of:
(a) Obtaining smashed raw materials as follows: 80.0-97.0% Radix Salviae Miltorrhizae, 1.0-19.0% Panax Notoginseng and 0.1-1.0 Borneol;
(b) Extracting the smashed Radix Salviae Miltorrhizae and Panax Notoginseng as obtained in step (a) in hot aqueous reflux at about 60-100°C in one or more extraction step;
(c) Filtering the extract as obtained in step (b) and combining the extracts in case of more than one extraction step (b) to form a combined extract;
(d) Concentrating the extract from step (c) to appropriate ratio of the volume of the concentrated extract to the weight of inputting herbal materials to form a concentrated extract;
(e) Putting ethanol into the concentrated extract from step (d) to about 50-85% final concentration of ethanol, performing ethanol precipitation and forming a precipitated resolution;
(f) Concentrating the supernatant liquid of precipitated resolution from step (e) to form a plaster of about 1.15-1.45 in relative density; and
(g) Mixing the plaster from step (f) with an appropriate amount of Borneol, thereby producing the composition of herb extract of Radix Salviae Miltorrhizae, Panax Notoginseng and Borneol.

According the process of the present invention, the raw materials comprise 90.0-97.0% Radix Salviae Miltorrhizae, 2.5-9.5% Panax Notoginseng and 0.2-0.5% Borneol. Preferably, the raw materials comprise 89.8% Radix Salviae Miltorrhizae, 9.6% Panax Notoginseng and 0.5% Borneol.

The process may comprise a further step of adding one or more kinds of excipients such as polyethylene glycol, xylitol, lactobacillus, starch for preparation of dripping pills, wherein the ratio of weight of the above total extracts or the above total pure ingredients to the weight of the excipient is 1:1-1:4.

The composition contains Sodium 3' 4-dihydroxyphenyllactate, 3' 4-dihydroxyphenyllactate, Protocatechuic Aldehyde, Salvianolic acid A, B, C, D, E, F, Rosemarinic acid, Ginsenoside Rg1, Ginsenoside Rbl, Ginsenoside Re, Notoginsenoside R1, Borneol, etc.
In another embodiment, the above composition further contains methyl tanshinonate, methyl rosmarinate, danshexinkum, lithospermic acid, d-bomeol, 1- borneol, tanshinone I, tanshinone IIA, tanshinone IIB, tanshinone V, tanshinone VI, isotanshinone, miltirone, dihydrotanshinone, 1-dehydrotanshinone and neocryptotanshinone, Salvianolic acid G and Salvianolic acid I, lithospermic acid B, ethyl lithospermate, dimethyl lithospermate, monomethyl lithospermate, ginsenoside Rd, ginsenoside Rg2, ginsenoside Rg3, ginsenoside Rh1 and ginsenoside Rh2, notoginsenoside R2, notoginsenoside R3, notoginsenoside R4, notoginsenoside R6, and notoginsenoside R7.

The process of the present invention may comprise a further step of formulated the composition in a dropping pellet, pill, capsule, granule, tablet, suspension, injection, syrup, tincture, powder, tea, topical solution, nebula, suppository microcapsule, or other pharmaceutically acceptable form.

This invention thus also provides a pill composition capable of treating chronic stable angina pectoris comprising about 0.14 to about 0.18 mg 3' 4-dihydroxyphenyllactate per pill, about 6.50 to about 40.50 mg Sanchinoside R1 per pill and about 25.60 to about 86.20 mg Ginsenoside Rg1 per pill.

### Quantitative analysis of 3' 4-dihydroxyphenyllactate in DSP

### Chromatography and systemic adaptive conditions, apparatus and reagents:

### Preparation of fingerprints

### (1) The parameters of Chromatogram & system adjustment.

Alkyl silan-linking silico-18 was used as a filling material, and water-acetonitrile-glacial acetic acid (87: 12: 1) as the mobile phase. Detective wavelength was set at 281 nm. The number of theoretical plates should not be less than 2,500 when calculated with the peak of Danshensu, and the degree of separation should meet the requirements.

### (2) Apparatus & reagents

Chromatograph: HP 1100 Liquid Chromatograph
Detector: HP VWD-stile ultraviolet detector
Column: Alltech Company 5u, 250 x 4.6 mm, ODS column
Pre-column: Alltech Company, Alltima C 18 5u pre-column
Temperature of the column: 30°C
Acetonitrile: chromatographically pure, Tianjin Siyou Biomedical & Technical Co. Ltd.
Glacial acetic acid: analytically pure, Tianjin Tianhe Reagent Company.

### (3) Preparation of the control sample.

Use 25.0 mg of 3' 4-dihydroxyphenyllactate as the control samples: Weigh 3' 4-dihydroxyphenyllactate and put it into the 50 ml measuring flasks. Add the mobile phase to dissolve them and dilute the solutions up to the scale line of the flasks. Shake them thoroughly and save them as stock solutions. Weigh a little amount of paraaminobenzoic acid accurately, dissolve it as a solution of 0.2 mg/ml with the mobile phase and take it as the internal standard stock solution. Pipette proper amounts of 3' 4-dihydroxyphenyllactate and internal standard solutions with their volumes accurately read, dilute them with the mobile phase to prepare a solution that contains 50 ug of 3' 4-dihydroxyphenyllactate and 80 ug of paraaminobenzoic acid. The prepared solution is taken as the control solution.

### (4) Preparation of the test sample.

Take 10 pills of above composition and 1 ml of internal standard stock solution, put them into a 25 ml measuring flask, dissolve them with the mobile phase, and dilute the solution to the scale line.

Take 10 µl of the control and the test sample solutions, respectively, make the injection, record the chromatograph chart and calculate the content.

The herbal composition comprising above composition should contain 0.14-0.18mg 3' 4-dihydroxyphenyllactate per pill.

### Quantitative analysis of Ginsenoside Rg1 and Sanchinoside R1 in DSP

### (1) Chromatographic system and system suitability

Alkyl silan-linking silico-18 was used as the stationary phase, and the mixture of water and acetonitrile as the mobile phase. The concentration of acetonitrile was 25% from 0 to 15 minutes, and 35% after 15th minute. Nebulizer gas flowrate was 2.5 liter per minute and drift tube temperature was set at 93.8°C. The number of theoretical plates of the column was not less than 5,000 when calculated with the peak of Ginsenoside Rg1.

### (2) Apparatus and reagents

Chromatograph: Agilent 1100 Liquid Chromatograph
Detector: Alltech ELSD 2000 detector (evaporative light scattering detector)
Column: Alltech Company 5u, 250 X 4.6mm, ODS-C₁₈ column
Pre-column: Alltech Company, Alltima C₁₈ 5u pre-column
Temperature of the column: 30°C

### (3) Preparation of the control sample

Ginsenoside Rg1 and Sanchinoside R1 are dissolved respectively in methanol to produce two control solutions each ml containing 0.98mg and 0.25mg correspondingly.

### (4) Preparation of the test sample

Put 50 pills of above composition into a 5 ml measuring flask, add 4% ammonia to the scale line, ultrasonicate for 20 minutes, and apply the solution to a previously prepare small C₁₈ column (STRATA C18-E column of Phenomenex Company, 500mg and 3ml tube), elute 10ml of water, discard the eluate, then elute 2ml of methanol, collect the eluate in a measuring flask and dilute it to the scale line with methanol, take the solution as the test solution.

### (5) Procedure

Accurately inject 10 µl each of the control solutions and the test solution, respectively, into the column, record the chromatograph chart and calculate the content.

This invention provides a composition for the treatment of cardiovascular and cerebrovascular disease comprising pure active ingredients extracted from *Salvia miltiorrhiza* Beg., *Panax notoginseng* or Ginseng, and *Dryobalanops aromatica Geartu.F.* or *Cinnammon camphor* or synthetic borneol . The active ingredient extracted from *Salvia miltiorrhiza* Beg. contains one or more ingredients selected from tanshinone, salvianolic acid, methyl tanshinonate, rosmarinic acid, methyl rosmarinate, danshexinkum, protocatechualdehyde, Sodium 3' 4-dihydroxyphenyllactate, lithospermic acid. The ingredient extracted from *Panax Notoginseng* or Ginseng contains one or more ingredients selected from notoginsenoside and ginsenoside. The ingredient extracted from *Dryobalanops aromatica Geartu.F.* or *Cinnammon camphor.* contains d-borneol or 1- borneol or both of them.

The said tanshinone includes tanshinone I, tanshinone IIA, tanshinone IIB, tanshinone V, tanshinone VI, isotanshinone, miltirone, dihydrotanshinone, 1-dehydrotanshinone, and neocryptotanshinone. The said salvianolic acid includes Salvianolic acid A, Salvianolic acid B, Salvianolic acid C, Salvianolic acid D, Salvianolic acid E, Salvianolic acid G, and Salvianolic acid I. The said lithospermic acid includes lithospermic acid B, ethyl lithospermate, dimethyl lithospermate, and monomethyl lithospermate. The said ginsenoside includes ginsenoside Rbl, ginsenoside Rd, ginsenoside Re, ginsenoside Rg1, ginsenoside Rg2, ginsenoside Rg3, ginsenoside Rh1, and ginsenoside Rh2. The said notoginsenoside includes notoginsenoside R1, notoginsenoside R2, notoginsenoside- R3, notoginsenoside R4, notoginsenoside R6, notoginsenoside R7.

This invention provides the above composition comprising Magnesium Salvianolate B or 3' 4-dihydroxyphenyllactate extracted from the *Salvia miltiorrhiza* Beg., Ginsenoside Rb₁ extracted from the *Panax notoginseng* or Ginseng, and d-Borneol extracted from the *Dryobalanops aromatica Geartu.F.* or *Cinnammon camphor.* In an embodiment, the composition comprises 10-80mg of Magnesium Salvianolate B, 10-50mg of Ginsenoside Rb₁ and 5-30mg of Borneol. In a further embodiment, the composition is characterized by comprising 50mg of Magnesium Salvianolate B, 20mg of Ginsenoside Rb₁ and 15mg of d-Borneol.

This invention provides the above composition, comprising 5-80mg of sodium 3' 4-dihydroxyphenyllactate, 10-50mg of Ginsenoside Rb₁ and 5-30mg of Borneol. In a further embodiment, this invention provides the above composition comprising 20mg of sodium 3' 4-dihydroxyphenyllactate, 20mg of Ginsenoside Rb₁ and 15mg of d-Borneol.

This invention provides the above composition further comprising one or more kinds of excipients such as polyethylene glycol, xylitol, lactobacillus, starch for preparation of dripping pills, wherein the ratio of weight the of above total extracts or above total pure ingredients to the weight of excipient is 1:1-1:4. For example, 50mg of Magnesium Salvianolate B, 20mg of Ginsenoside Rb₁, 15mg of d-Borneol and 265mg of excipient are contained per ten dripping pills; 20mg of sodium 3' 4-dehydrotanshinone, 20mg of Ginsenoside Rb₁ and 15mg of d-Borneol and 265mg of excipient are contained per ten dripping pills.

The Salvianolate B was extracted by steps of: Pulverizing *Salvia miltiorrhiza* Beg. into a fine powder, and decocting twice with hot water; Combining the decoctions, and concentrating under vacuum at about 50°C; Loading the solution from the step (b) into macroporous adsorption resin and, after washing with water, eluting column with 40% ethanol; After recovering the ethanol from solution obtained in the step (c), refining it by Sephadex LH-20 or other gel columns with the similar characteristics, eluting with ethanol and collecting eluant containing Magnesium Salvianolate B; Repeating the process of step (d) until the concentration of Magnesium Salvianolate B reaches more than 90%. The purity of concentration of Magnesium Salvianolate B was assayed by HPLC at a detective wavelength of 281 nm.

The Ginsenoside Rb₁ was extracted by steps of: Pulverizing *Panax notoginseng* or *Ginseng* into fine powder, and decocting with water; or refluxing with 70% ethanol; or percolating with 70% ethanol; Recovering solvent from the solution at reduced pressure; Loading the solution from the step (b) into macroporous adsorption resin, and after washing with water, eluting column with 40% ethanol; After recovering the ethanol from solution obtained in the step (c), refining it by silica gel column; Eluting the column with chloroform, methanol and water in the ratio of 6:3:1, respectively, and collecting eluant; Using TLC for examination of Ginsenoside Rb₁ and recovering the solvent, thereby obtaining Ginsenoside Rb₁.

This invention provides the above composition wherein d-Borneol was extracted from the trunk of *Dryobalanops aromatica Geartu.F.* or the leaves of *Cinnammon camphor.* by means of vapor distillation or supercritical carbon dioxide. In another embodiment, the Borneol is synthetic.

This invention also provides the above compositions, formulated in a dropping pellet, pill, capsule, granule, tablet, suspension, injection, syrup, tincture, powder, tea, topical solution, nebula, suppository microcapsule, or other pharmaceutically acceptable form.

In other words, this invention provides a pharmaceutical composition comprising the composition described herein and a pharmaceutically acceptable carrier. As known in the art, pharmaceutically acceptable carriers include 0.01-0.1M and preferably 0.05M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, chelating agents, inert gases and the like.

This invention provides compositions capable of increasing blood volume in the coronary artery, relaxing the smooth muscles of blood vessels, improving peripheral circulation, raising the oxygen content in veins, or significantly improving the acute myocardial ischemia or myocardial infarction, protecting the cells from damage by hypoxia or anoxia, protecting cells suffering from myocardial ischemia, improving micro-circulation, preventing arrhythmia, preventing platelet aggregation, and thrombosis, dissolving fibrin, lowering blood viscosity, adjusting blood cholesterol or preventing atherosclerosis, raising tolerance to hypoxia or anoxia, preventing the oxidation of lipoprotein or removing the harmful free radicals, lowering plasma ET content, significantly improving liver, kidney and pancreas function, preventing the occurrence or development of blood vessel or nerve diseases, enhancing the immune system, regulating vascular nerve balance.

This invention provides the above compositions, capable of preventing and treating cardiovascular and cerebrovascular disease, kidney disease, liver disease, pneumonia, lung or heart disease, pancreatitis, diabetes, vertebral disease, optic vessels disease, optic nerve disease, eccentric headache, chronic stomachitis, dizziness, bone disease, altitude disease, common diseases of the elderly, treating stable angina pectoris, unstable angina pectoris, angina pectoris of the aged, non-symptomatic myocardial ischemia, different types of coronary heart disease or angina pectoris disease, treating arrhythmia, enlargement of left ventricle, myocarditis, myocardial infarction or cerebral infraction, treating hyperlipidaemia, high blood viscosity syndrome or high blood pressure, treating diseases caused by micro-circulation disorder, treating stroke, cerebral infarction, cerebral bleeding and other cerebral diseases, treating hepatitis B, chronic liver fibrosis, liver fibrosis, active liver cirrosis, liver cirrosis in the remission period during recovery and other related diseases, treating kidney syndrome and its conjunctive diseases, treating diabetes or its conjunctive diseases, treating cyanosis-typed optic vessel diseases such as venal blockage in retina, central optic artery blockage in retina, high blood pressure optic atherosclerosis in retina, diabetic retinopathy, cento-neuropathy, cento-osmotic neuropathy, ischemic neuropathy, optic neuritis or optic nervous dystrophy, treating dizziness caused by cerebral-arterial ischemia, Meniere's disease, coronary heart disease, treating detrimental death of epicondylus medialis, femoral end necrosis, twisted joints, ligament damage, fracture and proliferation of bone cells, treating bronchitis in children, treating hypoxia or anoxia, treating Alzheimer's Disease.

The invention also describes a method of treatment using the above compositions for treating a specific disease. Effective amounts of the composition may be determined and administered to a subject suffering from said disease.

The subject invention further describes a method of treating a subject having a disorder, which comprises administering to the subject a therapeutically effective amount of the above composition to thereby treat the subject. In the preferred embodiment, the subject is a human.

The administration of the composition may be effected or performed using any of the various methods known to those skilled in the art. The route of administration includes administering intravenously, intramuscularly, intraperitoneally, and subcutaneously.

The therapeutically effective amount of the above compositions may be determined by methods well known to those skilled in the art.

### Animal Research

### 1. The effects of DSP on dogs with myocardial ischemia and myocardial infarction

Based on changes in myocardial oxygen consumption and biochemical standards, its pharmaceutical effects on curing coronary heart disease were investigated. DSP can significantly improve myocardial ischemia and myocardial infarction, raise the blood oxygen level of venous sinuses, inhibit the release of CK and LDH caused by damage to cardiac muscles, lower the activity of blood serum CK and LDH, suppress the activity of blood vessel substances, ET and TXB2, and raise the 6-Keto-PGF1/TXB2 ratio.

### 2. The protection of myocardial ischemical reperfusion injury from hypoxia in rats

This research stresses the effects of DSP in myocardial ischemical reperfusion injury from hypoxia in rats, especially on the apoptosis of myocardial cells. Results: Myocardial infarction did not occur after 7 hrs of sham operation. Myocardial hypoxia occurred for 1 hr and myocardial infarction occurred sharply after reperfusing for 6 hrs. DSP can reduce the M-IR area and increase the effects with increasing dosage.

### 3. Propagation of myocardial hypoxia in mice and effects of Fas/FasL proteins under deoxygenation and deoxygenation/re-oxygenation conditions

Fas gene is an apoptosis-stimulating gene. Its expressed protein product, Fas antigen, is a cell membrane protein. Recently, it was discovered, in experiments on propagation of myocardial hypoxia, that there is a close relationship between the expressed mRNA of Fas gene and myocardial apoptosis. FasL is the ligand of Fas. It is on the surface of transmembrane proteins, which is homologous to the TNF. It can bind to the receptor Fas on the surface of the cell and give out the death signal. The results show DSP can reduce apoptosis by interfering with Fas/FasL expression, protecting the cells from damage by hypoxia and deoxygenation/re-oxygenation.

### 4. Effects on lipidemia and atherosclerosis in rabbits

The testing showed that DSP can lower TC, TG, LDL-C, VLDL-C concentration, and TC/HDL-C ratio in the blood serum in rabbits. DSP also reduced the thickness of the aortic spot plate and the area of the aortic spot plates. DSP could adjust lipoprotein level and prevent atherosclerosis to a certain extent.

### 5. Anti-oxidation and removal of free radicals

By comparing the effect of Diltiazem and the effects of DSP on M-IR and its correlated biochemical markers, MDA and SOD can be observed. The SOD activity of the DSP group increased. There was an obvious difference when compared with the control group (P<0.01). DSP provides protection from injuries caused by reperfusion of the ischemic area and increases the activity of SOD. MDA is the main catabolic product of the oxidation of fats. It can damage the structure of the cell membrane so seriously that heart and liver tissue are damaged. SOD has an effective action of clearance on superoxide anions. It can regulate the oxidation reaction controlled by free radicals. DSP can increase SOD activity, decrease MDA content, lower the oxidation standard, and reduce the level of damage to the organs.

### 6. Effects on cardiac arrhythmia caused by exogenous free radicals

Langendorff perfusion device was used to pour ferrous sulfate (0.25mmol/L)/citrate (1.0mmol/L) into the Wistar rat's artificial heart. The model of free radicals causing heart rhythm irregularity was replicated to observe the effects of DSP. Exogenous free radicals can increase cardiac arrhythmia up to 100% and atrial cellular lysis up to 43%. 1mg/L verapamil and 50mg/L DSP can lower the irregularity percentage to 71.4% and 87.5%. It shows that DSP can prevent cardiac arrhythmia caused by free radicals.

### 7. Clinical applications for acute pancreatitis in rats

This experiment uses the acute pancreatitis model with multi-organ malfunction in rats to show the change in the blood plasma endothelins and the clinical applications of DSP. The experiment shows that blood plasma ET content increases significantly in acute pancreatitis with malfunction of multiple organs. Blood plasma ET content decreases significantly after DSP treatment, and treatment can greatly improve liver, kidney and pancreas function.

### 8. Prevention of platelet aggregation and thrombosis

The increasing of cAMP inhibits the activity of phosphoesterase and epoxidase, and reduces the production of prostaglandin peroxide. It can also activate protease to phosphoesterize the membrane protein, alter the effects of membrane protein composition on platelet aggregation, and control platelet aggregation to prevent thrombosis. DSP can increase blood platelet concentration and plasma camp content in order to prevent thrombosis.

### 9. Effects of DSP on blood vessels and nerve lesion in diabetic rats

DSP cannot thoroughly protect blood vessels and nerves or prevent the occurrence of blood vessel and nerve lesion in diabetic rats, but it can relieve or reduce its occurrence in the 6 month-diabetic rat, especially in terms of protein in the urine and lesion of blood capillaries of the kidneys and retina. This may be related to the function of DSP, which can increase thrombolysis.

### Clinical Research on Dan Shen Pill

### 1. Treatment of coronary heart disease with DSP

(1) Ordinary treatment of coronary heart disease with DSP
   After DSP came onto the market in China, a large-scale clinical research project was conducted in China. Although different prescriptions were used in different research projects, all clinical and experimental markers were standardized. The effects of DSP on the treatment of coronary heart disease are much better than that of Dan Shen tablet statistically. The treatment is basically similar to that of Isordil and there is no significant difference between them statistically. DSP works efficiently in small dosages. It is convenient, safe, easily absorbed, and has no side effects.
(2) Pain-killing effects of DSP on coronary heart disease compared with glyceryl trinitrate.
   The above experiment shows that the effects of DSP treatment on coronary heart disease is similar to that of glyceryl trinitrate. The results of both ECGs are similar 30 mins after treatment, and the Chinese classification of coronary heart disease does not affect the efficacy of DSP.
(3) Effects of DSP on the onset of coronary heart disease, heart pain frequency and volume of glyceryl trinitrate used.
   The results show that DSP can reduce onset frequency and volume of glyceryl trinitrate used. The level and duration of pain improved after a certain period of treatment, and the onset frequency also decreased. This explains why DSP can improve blood flow to the heart in addition to relieving pain.
(4) Improvement of blood pressure and cardiac function in patients with coronary heart disease.
   The results prove that DSP can improve cardiac function in patients with coronary heart disease and provide improvements in blood flow.
(5) Effects of DSP on ECGs and blood flow in patients with coronary heart disease
   No significant differences appeared on ECGs and average exercise testing standards to show improvement from DSP and Isordil (P>0.05), but the average exercise testing standards in the DSP group were much better before and after treatment (P< 0.01). This test proves that the treatment effects of DSP on coronary heart disease are the same as that of Isordil with no side effects and increase in tolerance. Also, DSP controls irregular blood flow, lowers blood viscosity, reduces the occurrence of atherosclerosis, and prevents thrombosis much better than Isordil and can be the first choice for the treatment of coronary heart disease.
(6) Effects of long-term DSP treatment on coronary heart disease
   Long-term DSP results are stable, and there is no antibiotic resistance. Isordil can efficiently lower blood pressure, leading to the activation of endogenous nerve and body fluid system and an increase in blood volume. In addition, Isordil works on sulfur radicals inside the capillary wall, but it would consume sulfur radicals in the long term and reduce treatment effects. DSP is a multi-level, multi-subjected and multi-method medicine which improves cardiac muscle; increases blood volume by blocking the chronic calcium route; stabilizes the myocardial membrane; removes free radials; regulates myocardial cells metabolism; improves blood platelets aggregation; and lowers cholesterol and blood viscosity. Therefore, long-term DSP treatment gives significant treatment effects.
(7) Clinical research on the effects of DSP on unstable-type angina.
   The experiment shows that DSP can reduce oxygen consumption by cardiac muscles, improve blood flow in coronary arteries, and rebalance the oxygen demand-to-oxygen supply ratio in cardiac muscles.
(8) The effects of DSP on the treatment of exertion-type angina
   DSP can efficiently relieve pain and increase blood flow to the cardiac muscle. DSP can also reduce oxygen consumption, improve blood flow to the coronary artery, rebalance oxygen demand and oxygen supply, and prevent atherosclerosis. It is the ideal medicine for the prevention or treatment of coronary heart disease, angina and atherosclerosis.
(9) Research on senior group angina
   Both DSP and Nifedipine can treat angina caused by coronary heart disease, but the latter has side effects which are not suitable for long-term use. In order to choose suitable drugs for patients with a need for long-term treatment of coronary heart disease, a comparative analysis of DSP and Nifedipine in the treatment of angina caused by coronary heart disease was carried out. DSP is made for angina. It activates blood circulation and relieves pain efficiently. Its effects are long lasting, require only a small dosage and have no side effects. Nifedipine is a short-term-effective calcium antagonist with a short half-life and functional time, so angina may occur during medication. It also has many side effects. Many reports state that long-term treatment with Nifedipine is harmful to coronary arteries. DSP can prevent decreased blood flow to the cardiac muscles and the development of atherosclerosis.

### 2. Effects on cardiac arrhythmia

The treatment effects of DSP on cardiac arrhythmia caused by coronary heart disease are significant. It is also helpful to those patients without heart disease. Its functions are: a) Calcification. DSP can reduce intracellular calcium concentration and prevent calcium overloading better than verapamil. b) Stabilizing the cell membrane. DSP can protect cardiac muscle and regulate heart rhythm. c) Removal of free radicals. d) Speeding up energy production and utilization. There is no relationship between chronic irregularity of heart rhythm and the addition of resistance and lack of energy supply.

DSP and Di'ao Xinxuekang can both improve cardiac arrhythmia caused by myocarditis and heart malfunction, but DSP does a better job than Di'ao Xinxuekang. DSP can improve blood flow to the cardiac muscles, section ST and T wave in ECGs. In addition, DSP can also reduce platelet aggregation, and platelet viscosity. The results show that patients who take DSP in the long term enjoy relief from symptoms and low reoccurrence of myocarditis.

### 3. Reverse function of DSP on Left Ventricular Hypertrophy (LVH)

The above experiment shows that DSP prevents damage caused by free radicals, prevents atherosclerosis, improves blood circulation, decreases blood viscosity and exterior blood vessel resistance, and regulates compliance of cardiac muscles to reverse LVH.

### 4. DSP treatment for high blood pressure

The experiment shows that DSP can stop and improve LVH and dilate the left ventricle, which can then lower blood pressure and combat angina.

Besides controlling blood pressure efficiently, other important steps for treating high blood pressure include: increasing the reactivity of insulin, lowering insulin level, and improving ET function in blood vessels. DSP is helpful because it also lowers blood pressure.

### 5. Treatment of hyperlipidaemia

This research shows that DSP can significantly lower blood lipoprotein level and improve blood flow, especially by the thinning of IMT after treatment. It explains how this drug can prevent atherosclerosis besides providing the above functions. DSP is safe and effective for older patients with coronary heart disease, angina and high blood viscosity.

### 6. Treatment for Hyperviscosity Syndrome (HS)

Hyperviscosity Syndrome (HS) is a pathobiological concept, a syndrome caused by one or more blood viscosity factor(s). It can lead to lack of blood supply, hypoxia, blocking, etc. in the heart, brain and kidneys. DSP offers the best results in HS treatment. After the normal 28 days of medication, HS symptoms such as nausea, lack of energy, breath holding, anxiety, etc. related to coronary heart disease, cerebral infraction, and kidney disease disappeared gradually. Blood pressure was lowered, and blood circulation improved. TC, TG, Apo-B dropped, and HDLC and Apo-A1 rose. All levels of hemorheology markers dropped. Renal Blood flow increased, and renal function improved. Urine protein decreased, and cardiac function improved.

### 7. Treatment for Acute Myocardial Infarction (AMI)

DSP can improve blood circulation by dilating coronary arteries, saving cardiac muscle, minimizing the infarction area, and protecting myocardial cells. Therefore, DSP can protect cardiac muscles in the early stages of AMI. It is a convenient medication with no side effects, which is recommended in the clinical field.

### 8. Effects on treatment of cerebral infarction

The use of DSP to treat low blood supply to the brain, cerebral infarction and internal bleeding is very efficient.

### 9. Effects on blood micro-circulation

The experimental results show that DSP can make an improvement in bulbar conjunctiva microcirculation and thrombo-elasticity in patients with coronary heart disease and angina.

### 10. Effects on immunity of red blood cells

This experiment uses the blood coagulation method of yeasts sensitized by complement, C_{3b} -causing yeast aggregation testing and Enzyme-linked Immunosorbent Assay (ELISA), to test the effects of DSP on the immunosorbent ability of red blood cells, CIC, and SIL-2R in patients with coronary heart disease. The experiment shows that DSP can lower SIL-2R level, strengthen the immune system and the immunosorbent ability of red blood cells.

### 11. Adjustment of vegetative nerve

This test uses "Wenger-Chongzhongchongxion" vegetative nerve balance factor analysis to test the heart rate variation (HRV), that is, the fluctuation in the average heartbeat over a certain period of time or over a long time period in the R-R period. Data, including effects on the sympathetic and para-sympathetic nerves can be calculated, to reflect the regulatory function of the vegetative nervous system. After DSP treatment, the percentage of y>+0.56 dropped significantly (P<0.05), but the drop in the Isordil group before and after treatment was not significant, y>+0.56 (P> 0.05). After DSP treatment, SDNN in R-R increased significantly (P<0.01), and there was no significant difference in the Isordil group before and after treatment (P >0.05). A decrease in HRV means the sympathetic nerve is excited. It is directly proportional to the symptoms in coronary heart disease and the possibility of sudden death and irregularity of heartbeat. DSP can control over-excitement of the para-sympathetic nerve and regulate the balance in the vegetative nerve.

### 12. DSP Treatment for liver disease

There is significant improvement in the treatment of hepatocirrhosis at the stage of losing compensation when DPS is added as a medication. DSP does not have side effects and is helpful in treating hepatocirrhosis.

### 13. The effectiveness of DSP therapy in diabetes and related complications

Trial results reveal that after 3 months of taking DSP, the 40 patients' nail wall microcirculation test indexes had varying degrees of improvement. Their collective cumulative values decreased. Among the patients, those originally with serious abnormalities now had medium abnormality; those originally with medium abnormality had slight abnormalities. The differences before and after treatment were obvious. DSP has positive healing effects on diabetic end-brush neuritis.

### 14. The effectiveness of DSP therapy on optical fundus vascular diseases

The cause of retinal vein occlusion is still not very clear. Hypertention, hyperlipidemia, and arteriosclerosis are usually considered as likely causes of retinal vein occlusion. Doctors of traditional Chinese medicine believe that it is caused by stagnant blood flow. DSP can activate blood circulation and relieve congestion, improve microcirculation, relieve hydropsy, and encourage blood absorption. In so doing, it can improve eyesight. DSP can also be used to heal different kinds of optical fundus vascular diseases that are generally termed as Xueyuzheng, such as central retinal artery occlusion, hypertensive retinal arteriosclerosis, diatetic retinal lesion, central plasm optic neuropathy, central permeation optic neuropathy, ischemic optic neuropathy, optic neuritis, atrophy of the optic nerve, etc.

### 15. DSP's effect on hemorheology

In the Observation group, besides fibrinogen, whole blood viscosity, whole blood reduction viscosity, plasma viscosity, hematocrit and Aggregation Index of RBC all decreased. The differences before and after treatment were obvious (P<0.05 or P<0.01). Compared with the Control group, whole blood viscosity, whole blood reduction viscosity, plasma viscosity, and Aggregation Index of RBC had obvious decreases (P<0.05 or P<0.01). After treatment, the Control group shared an obvious change only in the hemagglutilation index (P<0.05 or P<0.01).

### 16. DSP's healing effect on chronic pulmonary heart disease

The total efficacy rate of treatment group is 95% and that of the control group 76%, the difference of which is statistically significant (P<0.05). The result revealed that the efficacy of DSP is superior to persantin and that hemorheology in the DSP group has more improvement than that in the persantin group. There was a significant difference in the efficacy of the two groups (X²=4.46 and 4.95, P<0.05). Treatment group results were better than that of the Control group. There were obvious changes in blood flow in the Treatment group before and after treatment compared with that of the Control group (P<0.05). The blood viscosity of the control group decreased after treatment, but there was no statistical deviation.

### 17. Treatment of adrenal syndrome

The results show that the complete reversal percentage and total efficacy in the treatment group were 55% and 90%, respectively, which was significantly higher than those in the control group: 27.5% and 65% (P<0.05). DSP, combined with other medications, can improve treatment results and reduce the percentage of reoccurrence.

### 18. Treatment of other diseases

(1) Treatment of bronchitis in children
   DSP combined with antibiotics can improve treatment results in infections. The DSP treatment ended fever and rales better than that of the control group. DSP raised the recovery percentage for pneumonia in children and shortened the duration of treatment without significant side effects.
(2) Effects on hemicrania. 58 patients with hemicrania were selected from clinics. The result shows that the efficacy in the treatment group is higher than that of the control group (P<0.05). DSP can efficiently cure and prevent hemicrania.
(3) Treatment of chronic gastritis
   DSP can regulate the function of blood vessels, restrain platelet aggregation, control thrombosis, clear out stagnant blood in the gastric mucosa and cure stomachache caused by chronic gastritis. It can efficiently eliminate the dead parts of the erosive gastric mucinitis, activate megakaryocytes, and stimulate production of new cells to improve the recovery from inflammation.
(4) Treatment of dizziness
   The total efficacy in the Treatment group and the Control group were 86% and 87.5%, respectively. No significant abnormality was found. These two groups of drugs can treat fainting efficiently. Therefore, DSP can be a convenient and efficient drug to treat fainting caused by insufficient blood supply to the brain.
(5) Treatment of damaged lateral malleolus joint
   DSP can eliminate swelling and stagnant blood, and can kill pain. One active ingredient, borneol, can increase DSP's absorption through the skin and maintain concentrations of the drug at the application site, so it can efficiently and quickly treat damage of the lateral malleolus joint. It is helpful in treating broken bones, bone death and proliferation of bone.
(6) Prevention and treatment of plateau hypoxia
   Plateau hypoxia can lead to capillary circulation disorder, causing blood-perfusing insufficiency. Plateau hypoxia also leads to high blood viscosity, increased red blood cell quantity-and red blood cell aggregation, enhanced red blood cell rigidity, increased platelet aggregation, and change in pH value. All the above factors affect blood viscosity and the radius of capillaries. Platelet aggregation can increase resistance in capillaries, leading to blockage. When blood viscosity increases, the radius of capillaries also increases and leads to increased resistance and congestion. There are common properties in the blood flow of people with plateau hypoxia: "concentration" (increased red blood cell pressure), "viscosity" (increased whole blood viscosity), and "aggregation" (increased aggregation of red blood cells). All the above are different at different sea levels and durations. Forementioned pharmaphysiologic and clinical research show that DSP can lower hematocrit, blood sedimentation and blood viscosity, so it is helpful in preventing and treating plateau hypoxia.
(7) Prevention and treatment of Senile dementia
   Senile dementia can be classified as Alzheimer's Disease (AD), vascular dementia and combined dementia. After DSP treatment, there is statistically significant improvement in AD and vascular dementia by measurement analysis and Chinese medicine clinical observation (p<0.05 or p<0.01). DSP is helpful for treating sluggishness, reticence, forgetfulness, fatigue, and ecchymosis at a total efficacy of 40%, and sadness, anger, rashness, and irritation at a total efficacy of 85.7%.

This invention will be better understood from the examples which follow. However, one skilled in the art will readily appreciate that the specific methods and results discussed are merely illustrative of the invention as described more fully in the claims which follow thereafter.

### EXAMPLE 1

41.06g of Radix Salviae Miltiorrhizae, 8.03g of Radix Notoginseng were respectively weighed and ground, and then sequentially introduced into an extractor. 5 parts water were added, the mixture was decocted for 2 hours, filtered, and the first filtrate was removed. The residue was added to 4 parts water, decocted for 1 hour, filtered, and then this second filtrate was mixed with the first filtrate. The mixed filtrate was concentrated under decompressed conditions until the solution volume (L) to raw materials weight (Kg) ratio was 0.9-1.1. 95% ethanol was gradually poured in until the concentration of ethanol was 69-71 %, settled for 12 hours, and filtered. The filtrate, in which ethanol is evaporated, is concentrated to extract of the relative of 1.32-1.40.

The above extract was mixed with 0.46g of borneol and 18 g of polyethylene glycol 6000. The mixture was heated to 85°C, melted for 30 minutes, and then transferred to a dropping machine at 80°C . The melted mixture was dropped into the liquid paraffin, at a temperature of 7°C . The dropping pills were taken out, and the oil was removed.

The dropping pill is a reddish brown-brownish black sphere with an even size, smooth color, scent, and bitter taste. The weight per pill is 25mg ± 15%, and the diameter per pill is 3.34 ± 15% mm.

The above dropping pill contains the following active ingredients: Sodium 3' 4-dihydroxyphenyllactate, Protocatechuic Aldehyde, Salvianolic acid A, B, C, D, E, F, Rosemarinic acid, Ginsenoside Rg1, Ginsenoside Rb1, Ginsenoside Re, Notoginsenoside R1, Borneol, etc.

### EXAMPLE 2

31.12g of Radix Salviae Miltiorrhizae, 9.21g of Radix Notoginseng, 0.50g of borneol and 20g of polyethylene glycol 6000 were taken and processed according to the extraction and preparation method of Example 1, except for the difference in parameters as follows: the temperature of the dropping machine was 64°C, and the temperature of the liquid paraffin was 0°C .

The dropping pill is a reddish brown-brownish black sphere with an even size, smooth color, distinct scent, and bitter taste. The weight per pill is 25mg± 15%, and the diameter per pill is 3.34 ± 15% mm.

The above dropping pill contains the following 5 active ingredients: Sodium 3' 4-dihydroxyphenyllactate, Protocatechuic Aldehyde, Salvianolic acid A, B, C, D, E, F, Rosemarinic acid, Ginsenoside Rg1, Ginsenoside Rbl, Ginsenoside Re, Notoginsenoside R1, Borneol, etc.

### EXAMPLE 3

59.36g of Radix Salviae Miltiorrhizae, 6.38g of Radix Notoginseng, 0.34g of borneol and 21 of polyethylene glycol 6000 were taken and processed according to the extraction and preparation method of Example 1, except for the difference in parameters as follows: the temperature of the dropping machine was 69°C, and the temperature of polymethyl siloxane as condensation liquid was 4°C.

The dropping pill is a reddish brown-brownish black sphere with an even size, smooth color, distinct scent, and bitter taste. The weight per pill is 25mg± 15%, and the diameter per pill is 3.86 ± 15% mm.

The above dropping pill contains the following active ingredients: Sodium 3' 4-dihydroxyphenyllactate, Protocatechuic Aldehyde, Salvianolic acid A, B, C, D, E, F, Rosemarinic acid, Ginsenoside Rg1, Ginsenoside Rbl, Ginsenoside Re, Notoginsenoside R1, Borneol, etc.

### EXAMPLE 4

59.36g of Radix Salviae Miltiorrhizae, 6.38g of Radix Notoginseng, 0.34g of borneol, 40g of xylitol and 8g of starch were taken and processed according to the extraction and preparation method of Example 1, except for the difference in parameters as follows: the temperature of dropping machine was at 69°C , the temperature of polymethyl siloxane as condensation liquid was at 4°C .

The dropping pill is a reddish brown-brownish black sphere with an even size, smooth color, distinct scent, and bitter taste. The weight per pill is 35mg± 15%, and the diameter per pill is 3.86 ± 15% mm.

The above dropping pill contains following active ingredients: Sodium 3' 4-dihydroxyphenyllactate, Protocatechuic Aldehyde, Salvianolic acid A, B, C, D, E, F, Rosemarinic acid, Ginsenoside Rg1, Ginsenoside Rb1, Ginsenoside Re, Notoginsenoside R1, Borneol, etc.

### REFERENCE EXAMPLE 5

50mg of Magnesium Salvianolate B, 20mg of Ginsenoside Rb₁, 15mg of d-Borneol and 265mg of polyethylene glycol 6000 were mixed and processed according to extraction and preparation method of Example 1, except for the difference in parameters as follows: the temperature of the dropping machine was 64°C, and the temperature of the liquid paraffin was 4°C.

### REFERENCE EXAMPLE 6

80mg of Magnesium Salvianolate B, 10mg of Ginsenoside Rb₁, 10mg of d-Borneol and 245mg of polyethylene glycol 6000 were mixed and processed according to the extraction and preparation method of Example 1, except for the difference in parameters as follows: the temperature of dropping machine was 69 °C , the temperature of the liquid paraffin was 5°C .

### REFERENCE EXAMPLE 7

60mg of Magnesium Salvianolate B, 25mg of Ginsenoside Rb₁, 8mg of d-Borneol, 200g of xylitol and 48g of starch were mixed, and processed according to the extraction and preparation method of Example 1, except for the difference in parameters as follows: the temperature of the dropping machine was 80°C, and the temperature of polymethyl siloxane as condensation liquid was 4°C.

### REFERENCE EXAMPLE 8

30mg of sodium3' 4-dihydroxyphenyllactate, 40mg of Ginsenoside Rb₁, 15mg of d-Borneol, 180g of lactobacillus and 58g of starch were mixed and processed according to the extraction and preparation method of Example 1, except for the difference in parameters as follows: the temperature of the dropping machine was 80°C, and the temperature of liquid paraffin was 4°C .

### REFERENCE EXAMPLE 9

50mg of sodium3' 4-dihydroxyphenyllactate, 30mg of Ginsenoside Rb₁, 10mg of d-Borneol and 250g of polyethylene glycol 6000 were mixed and processed according to the extraction and preparation method of Example 1, except for the difference in parameters as follows: the temperature of the dropping machine was 80°C, and the temperature of the liquid paraffin was 4°C.

### REFERENCE EXAMPLE 10

40mg of sodium3' 4-dihydroxyphenyllactate, 25mg of Ginsenoside Rb₁, 8mg of d-Borneol, 200g of lactobacillus and 35g of starch were mixed and processed according to the extraction and preparation method of Example 1, except for the difference in parameters as follows: the temperature of the dropping machine was 80°C, and the temperature of polymethyl siloxane as condensation liquid was 4°C.

### REFERENCE EXAMPLE 11

25mg of sodium 3' 4-dihydroxyphenyllactate, 45mg of Ginsenoside Rb₁, 12mg of d-Borneol, 185g of xylitol and 30g of starch were mixed and processed according to the extraction and preparation method of Example 1, except for the difference in parameters as follows: the temperature of the dropping machine was 80°C, and the temperature of polymethyl siloxane as condensation liquid was 4°C.

### REFERENCE EXAMPLE 12

20mg of methyl tanshinonate, 15mg of ginsenoside Rd, 35mg of ginsenoside Re, 15mg of d-Borneol, 245mg of polyethylene glycol 6000 were mixed and processed according to the extraction and preparation method of Example 1, except for the difference in parameters as follows: the temperature of the dropping machine was 80°C, and the temperature of polymethyl siloxane as condensation liquid was 4°C.

### REFERENCE EXAMPLE 13

10mg of rosmarinic acid, 15mg of methyl rosmarinate, 20mg of tanshinone IIA, 25mg of ginsenoside Rg2, 15mg of d-Borneol, 215g of lactobacillus and 30g of starch were mixed and processed according to the extraction and preparation method of Example 1, except for the difference in parameters as follows: the temperature of the dropping machine was 82°C , and the temperature of polymethyl siloxane as condensation liquid was 4°C.

### REFERENCE EXAMPLE 14

12mg of Salvianolic acid C, 15mg of Salvianolic acid E, 20mg of ginsenoside Rh1, 15mg of ginsenoside Rh2, 15mg of d-Borneol, 210g of lactobacillus and 30g of starch were mixed and processed according to the extraction and preparation method of Example 1, except for the difference in parameters as follows: the temperature of the dropping machine was 80 °C , and the temperature of polymethyl siloxane as condensation liquid was 4°C.

### REFERENCE EXAMPLE 15

8mg of Salvianolic acid D, 10mg of Salvianolic acid G, 15mg of Salvianolic acid I, 30mg of ginsenoside Rg3, 15mg of d-Borneol, 245mg of polyethylene glycol 6000 were mixed and processed according to the extraction and preparation method of Example 1, except for the difference in parameters as follows: the temperature of the dropping machine was 79 °C , and the temperature of the liquid paraffin as condensation liquid was 4°C.

### REFERENCE EXAMPLE 16

8mg of tanshinone IIB, 15mg of tanshinone I, 8mg of dimethyl lithospermate, 15mg of protocatechualdehyde, 15mg of notoginsenoside R2, 10mg of notoginsenoside- R3, 15mg of d-Borneol, 210g of lactobacillus and 30g of starch were mixed and processed according to the extraction and preparation method of Example 1, except for the difference in parameters as follows: the temperature of the dropping machine was 85°C, and the temperature of polymethyl siloxane as condensation liquid was 4°C.

### REFERENCE EXAMPLE 17

8mg of tanshinone V, 12mg of isotanshinone, 8mg of monomethyl lithospermate, 10mg of notoginsenoside R4, 25mg of ginsenoside Rg1, 15mg of 1-Borneol, 210g of xylitol and 30g of starch were mixed and processed according to the extraction and preparation method of Example 1, except for the difference in parameters as follows: the temperature of the dropping machine was 80 °C , and the temperature of polymethyl siloxane as condensation liquid was 4°C.

### REFERENCE EXAMPLE 18

8mg of tanshinone VI, 5mg of 1-dehydrotanshinone, 5mg of neocryptotanshinone, 15mg of ethyl lithospermate, 10mg of notoginsenoside R6, 15mg of Salvianolic acid A, 15mg of d-Borneol, 245mg of polyethylene glycol 6000 were mixed and processed according to the extraction and preparation method of Example 1, except for the difference in parameters as follows: the temperature of the dropping machine was 80°C, and the temperature of the liquid paraffin as condensation liquid was 4°C.

### REFERENCE EXAMPLE 19

25mg of lithospermic acid B, 15mg of dihydrotanshinone, 40mg of ginsenoside Rb1, 10mg of notoginsenoside R7, 15mg of d-Borneol, 215g of xylitol and 30g of starch were mixed and processed according to the extraction and preparation method of Example 1, except for the difference in parameters as follows: the temperature of the dropping machine was 80 °C , and the temperature of polymethyl siloxane as condensation liquid was 4°C.

### REFERENCE EXAMPLE 20

Magnesium Salvianolate B was extracted by steps of:
(1) Pulverizing *Salvia miltiorrlviza* Beg. into a fine powder and decocting twice with hot water;
(2) Combining the decoctions and concentrating in vacuum under 50 °C;
(3) Loading the solution from the step (2) into macroporous adsorption resin and, after washing with water, eluting column with 40% ethanol;
(4) After recovering the ethanol from solution obtained in the step (3), refining it by Sephadex LH-20 or other gel columns with similar characteristics, eluting with ethanol and collecting eluant containing Magnesium Salvianolate B.
(5) Repeating the process of step (4) until the concentration of Magnesium Salvianolate B reaches more than 90%.

### REFERENCE EXAMPLE 21

Ginsenoside Rb₁ was extracted by steps of:
(1) Pulverizing *Panax notoginseng* or *Ginseng* into a fine powder, and decocting with water; or refluxing with 70% ethanol; or percolating with 70% ethanol;
(2) Recovering solvent from the solution at reduced pressure;
(3) Loading the solution from step (2) into macroporous adsorption resin and, after washing with water, eluting column with 40% ethanol;
(4) After recovering the ethanol from the solution obtained in step (3), refining it by silica gel column;
(5) Eluting the column with chloroform, methanol and water in the ratio of 6:3: 1, respectively, and collecting the eluant;
(6) Using TLC for examination of Ginsenoside Rb₁ and recovering the solvent, thereby obtaining Ginsenoside Rb₁.

### REFERENCE EXAMPLE 22

6mg of tanshinoneII A, 10mg of Salvianolic acid A, 8mg of lithospermic acid B, 25mg of ginsenoside- Rg1, 12mg of d-Borneol, 40g of microcrystalline cellulose, 0.5g of tale powder and appropriate amount of 3% polyvidone in ethanol solution were taken and made into tablets according to standard procedures.

### REFERENCE EXAMPLE 23

10mg of rosmarinic acid, 30mg of sodium 3' 4-dihydroxyphenyllactate, 10mg of ginsenoside- Rg3, 20mg of notoginsenoside- R1, 8mg of d-Borneol, 50g of gelatin and 10g of glycerin were taken and made into capsules according common practices.

### REFERENCE EXAMPLE 24

Salvianolic acid A, ethyl lithospermate, ginsenoside- Re, notoginsenoside- R3, 10m of d-Borneol, 30g of magnesium stearate, 15g of starch and an appropriate amount of 3% polyvidone in ethanol solution were taken and made into granules according to routine procedures.

### REFERENCE EXAMPLE 25

10mg of lithospermic acid, 20mg of sodium 3' 4-dihydroxyphenyllactate, 20mg of ginsenoside- Rg3, 8mg of d-Borneol, 35g of microcrystalline cellulose, 10g of starch and an appropriate amount of 3% polyvidone in ethanol solution were taken and made into pills according to routine procedures.

### REFERENCE EXAMPLE 26

60mg of Salvianolic acid B, 10mg of sodium 3' 4-dihydroxyphenyllactate, 20mg of Ginsenoside Rb₁, 10mg of d-Borneol, 30mg of mannitol and 5mg of antallin and an appropriate amount of water were taken and made into a sterile powder for treating infection according to routine procedures.

## Claims

1. A process for preparing a composition for treatment of chronic stable angina pectoris, **characterized in** comprising the steps of:
(a) Obtaining smashed raw materials as follows: 80.0-97.0% Radix Salviae Miltiorrhizae, 1.0-19.0% Panax Notoginseng and 0.1-1.0% Borneol;
(b) Extracting the smashed Radix Salviae Miltiorrhizae and Panax Notoginseng as obtained in step (a) in hot aqueous reflux at about 60-100°C in one or more extraction step;
(c) Filtering the extract as obtained in step (b) and combining the extracts in case of more than one extraction step (b) to form a combined extract;
(d) Concentrating the extract from step (c) to appropriate ratio of the volume of the concentrated extract to the weight of inputting herbal materials to form a concentrated extract;
(e) Putting ethanol into the concentrated extract from step (d) to about 50-85% final concentration of ethanol, performing ethanol precipitation and forming a precipitated resolution;
(f) Concentrating the supernatant liquid of precipitated resolution from step (e) to form a plaster of about 1.15-1.45 in relative density; and
(g) Mixing the plaster from step (f) with an appropriate amount of Borneol, thereby producing the composition of herb extract of Radix Salviae Miltiorrhizae, Panax Notoginseng and Borneol.

2. The process according to claim 1, wherein the raw materials is 90.0-97.0% Radix Salviae Miltiorrhizae, 2.5-9.5% Panax Notoginseng and 0.2-0.5% Borneol.

3. The process according to claim 1, wherein the raw materials is 89.8% Radix Salviae Miltiorrhizae, 9.6% Panax Notoginseng and 0.5% Borneol.

4. The process of any one of the above claims comprising a further step of adding one or more kinds of excipients such as polyethylene glycol, xylitol, lactobacillus, starch for preparation of dripping pills, wherein the ratio of weight of the above total extracts or the above total pure ingredients to the weight of the excipient is 1:1-1:4.

5. The process of any one of the above claims comprising a further step of formulated the composition in a dropping pellet, pill, capsule, granule, tablet, suspension, injection, syrup, tincture, powder, tea, topical solution, nebula, suppository microcapsule, or other pharmaceutically acceptable form.

## Patentansprüche

1. Verfahren zur Zubereitung einer Zusammensetzung zur Behandlung einer chronischen stabilen Angina Pectoris, **dadurch gekennzeichnet, dass** es die folgenden Schritte umfasst:
(a) Erhalten von gebrochenen Rohmaterialien wie folgt: 80,0 - 97,0 % Radix Salviae Miltiorrhizae, 1,0 - 19,0 % Panax Notoginseng und 0,1 - 1,0 % Borneol;
(b) Extrahieren des gebrochenen Radix Salviae Miltiorrhizae und Panax Notoginseng wie in Schritt (a) erhalten in einem heißen wässrigen Rückfluss bei ungefähr 60 - 100 °C in einem oder in mehreren Extraktionsschritten;
(c) Filtern des Extrakts, wie in Schritt (b) erhalten, und Kombinieren der Extrakte im Fall von mehr als einem Extraktionsschritt (b), um einen kombinierten Extrakt zu bilden;
(d) Konzentrieren des Extrakts von Schritt (c) auf das geeignete Verhältnis des Volumens des konzentrierten Extrakts zum Gewicht der Eingabe von pflanzlichen Materialien, um einen konzentrierten Extrakt zu bilden;
(e) Hinzugeben von Ethanol in den konzentrierten Extrakt von Schritt (d) zu einer endgültigen Konzentration von Ethanol von ungefähr 50 - 85 %, Durchführen einer Ethanol-Ausfällung und Bilden einer ausgefällten Auflösung,
(f) Konzentrieren der überstehenden Flüssigkeit der ausgefällten Auflösung von Schritt (e), um ein Pflaster von ungefähr 1,15 bis 1,45 relativer Dichte zu bilden; und
(g) Mischen des Pflasters von Schritt (1) mit einer angemessenen Menge von Borneol, wodurch die Zusammensetzung des pflanzlichen Extrakts aus Radix Salviae Miltiorrhizae, Panax Notoginseng und Borneol hergestellt wird.

2. Verfahren nach Anspruch 1, wobei die Rohmaterialien 90,0 - 97,0 % Radix Salviae Miltiorrhizae, 2,5 - 9,5 % Panax Notoginseng . und 0,2 - 0,5 % Borneol sind.

3. Verfahren nach Anspruch 1, wobei die Rohmaterialien 89,8 % Radix Salviae Miltiorrhizae, 9,6 % Panax Notoginseng und 0,5 % Borneol sind.

4. Verfahren nach einem der vorhergehenden Ansprüche, umfassend einen weiteren Schritt des Hinzugebens von einer oder mehrere Arten von Trägerstoffen wie z.B. Polyethylenglykol, Xylitol, Lactobazillus, Stärke für die Zubereitung von Dripping Pills, wobei das Verhältnis des Gewichts der oben angegebenen gesamten Extrakte oder der oben angegebenen reinen Inhaltsstoffe zum Gewicht des Trägerstoffs 1:1-1:4 ist.

5. Verfahren nach einem der vorhergehenden Ansprüche, umfassend einen weiteren Schritt des Formulierens der Zusammensetzung in eine Tropfentablette, eine Pille, eine Kapsel, ein Körnchen, eine Tablette, eine Suspension, eine Injektion, einen Sirup, eine Tinktur, ein Pulver, einen Tee, eine topische Lösung, einen Nebel, ein Mikrokapsel-Suppositorium oder andere pharmazeutisch akzeptable Formen.

## Revendications

1. Procédé de préparation d'une composition pour le traitement de l'angine de poitrine chronique stable, **caractérisé en ce qu'**il comprend les étapes :
(a) d'obtention de matériaux bruts broyés comme suit : 80,0 à 97,0 % de Radix Salviae Miltiorrhizae, 1,0 à 19,0 % de Panax Notoginseng et 0,1 à 1,0 % de bornéol ;
(b) d'extraction de Radix Salviae Miltiorrhizae broyé et de Panax Notoginseng obtenus à l'étape (a) à reflux aqueux chaud à environ 60 à 100° C en une ou plusieurs étape(s) d'extraction ;
(c) de filtrage de l'extrait obtenu à l'étape (b) et d'association des extraits dans le cas de plus d'une étape d'extraction (b) pour former un extrait combiné ;
(d) de concentration de l'extrait de l'étape (c) à un rapport approprié du volume de l'extrait concentré au poids de matériaux herbacés intégrés pour former un extrait concentré ;
(e) d'intégration d'éthanol dans l'extrait concentré de l'étape (d) à environ 50 à 85 % de concentration finale d'éthanol, de réalisation d'une précipitation à l'éthanol et de formation d'une résolution précipitée ;
(f) de concentration du liquide surnageant de la résolution précipitée de l'étape (e) pour former un emplâtre d'environ 1,15 à 1,45 en densité relative ; et
(g) de mélange de l'emplâtre de l'étape (f) avec une quantité appropriée de bornéol, produisant ainsi la composition d'extrait herbacé de Radix Salviae Miltiorrhizae, de Panax Notoginseng et de bornéol.

2. Procédé selon la revendication 1, dans lequel le matériau brut comprend 90,0 à 97,0 % de Radix Salviae Miltiorrhizae, 2,5 à 9,5 % de Panax Notoginseng et 0,2 à 0,5 % de bornéol.

3. Procédé selon la revendication 1, dans lequel le matériau brut comprend 89,8 % de Radix Salviae Miltiorrhizae, 9,6 % de Panax Notoginseng et 0,5 de bornéol.

4. Procédé selon l'une quelconque des revendications précédentes, comprenant une étape supplémentaire d'addition d'une ou plusieurs sortes d'excipients tels que le polyéthylène glycol, le xylitol, des lactobacilles, de l'amidon, pour la préparation de pilules à matrice de diffusion, dans lequel le rapport en poids des extraits totaux ci-dessus ou des ingrédients purs totaux ci-dessus au poids de l'excipient est de 1:1 à 1:4.

5. Procédé selon l'une quelconque des revendications précédentes, comprenant une autre étape consistant à formuler la composition en une pastille à matrice de diffusion, une pilule, une capsule, un granule, un comprimé, une suspension, un produit pour injection, un sirop, une teinture, une poudre, une infusion, une solution topique, une pulvérisation, un suppositoire, une microcapsule ou une autre forme pharmaceutiquement acceptable.
